# EUROPÄISCHE PATENTSCHRIFT

(11) **EP 2 549 976 B1**
(45) Veröffentlichungstag und Bekanntmachung des Hinweises auf die Patenterteilung: **23.11.2016**
(21) Anmeldenummer: 11711294.6
(22) Anmeldetag: 16.03.2011
(51) Int. Cl.: A61K 8/06, A61K 8/35, A61K 8/40, A61K 8/49, A61K 8/97, A61Q 17/04, A61K 8/34, A61K 8/44, A61Q 19/00

(54) **HAUTFREUNDLICHE WIRKSTOFFKOMBINATION GEGEN AKNE**
SKIN-FRIENDLY ACTIVE INGREDIENT COMBINATION TO COMBAT ACNE
ASSOCIATION DE PRINCIPES ACTIFS RESPECTUEUSE DE LA PEAU POUR LUTTER CONTRE L'ACNÉ

(30) Priorität: 22.03.2010 DE 102010012384
(43) Veröffentlichungstag der Anmeldung: 30.01.2013
(73) Patentinhaber: Beiersdorf AG, 20253 Hamburg (DE)
(72) Erfinder: KURZ, Nadeshda, 22045 Hamburg (DE); KRÖPKE, Rainer, 22869 Schenefeld (DE); HIDDEMANN, Sarah, 20251 Hamburg (DE); NEUFANG, Gitta, 20149 Hamburg (DE); THIESEN, Kathrin, 22303 Hamburg (DE); SCHLÄGER, Torsten, 22297 Hamburg (DE); PEIRANO, Reto, 22761 Hamburg (DE)
(74) Vertreter: Hartmann, Jost
(86) Internationale Anmeldenummer: PCT/EP2011/053959
(87) Internationale Veröffentlichungsnummer: WO 2011/117126

(56) Entgegenhaltungen:
- WO-A1-2005/030157
- WO-A1-2007/135083
- WO-A1-2008/046791
- WO-A1-2008/135085
- DE-A1- 10 341 179
- DE-A1-102007 022 449

## Beschreibung

Die vorliegende kosmetische oder dermatologische Zubereitung umfasst eine Kombination aus 1,2-Decandiol und Licochalcone A sowie Carnitin und ist gegen Propionibacterium acnes (P. acnes) wirksam ohne Haut irritierend zu wirken.

Der Begriff "unreine, zu Akne neigende Haut" beschreibt Hautveränderungen wie Fettglanz und Komedonen, die auf der Basis eines seborrhoischen Hautzustandes entstehen können. Unreine Haut und Akne zeigen sich im Wesentlichen an talgdrüsenreichen Hautarealen wie Gesicht, vorderer Brustwand, Schultern und Rücken. Die häufigste Akneform, die Acne vulgaris oder simplex, tritt in der Regel während der Pubertät auf. Von unreiner und seborrhoischer Haut sind jedoch zunehmend auch junge Erwachsene betroffen.

Seborrhoe (auch genannt Seborrhoea oder Schmerfluss) steht für die Überproduktion von Sebum (Talg) in einer Talgdrüse. Die Überproduktion an Sebum verursacht, dass die Talgdrüsenfollikel mit Sebum teilweise oder vollständig angefüllt sind. Dieses Sebum weist oftmals eine sehr feste Konsistenz und demzufolge eine niedrige Spreitung auf, so dass es nur sehr schwierig aus dem Talgdrüsenfollikel freigesetzt wird und sich auf der Haut ausbreiten kann. In Folge dessen reichert sich das Sebum in unerwünscht hohem Maße in den Talgdrüsen an und bildet einen idealen Nährboden für Mikroorganismen, wie insbesondere Propionibacterium acnes, einem bei der Entstehung von Akne maßgeblich beteiligten Mikroorganismus.

Die primären Symptome der unreinen, fettigen Haut bzw. Akne sind vermehrt produziertes Hautfett (Sebum) und Komedonen ("Mitesser"). Durch eine Verhornungsstörung verstopfen die Follikel und es kommt zu einem Talgrückstau. Man unterscheidet so genannte offene "blackheads" von geschlossenen "whiteheads". Papeln (Knötchen), Nodi (Knoten) und Pusteln (Eiterbläschen) können entstehen. Durch die starke Vermehrung von Aknebakterien (Propionibacterium acnes) in den Komedonen kommt es zu Entzündungserscheinungen im umliegenden Gewebe.

Bei den Staphylokokken handelt es sich um grampositive Kugelbakterien, die in unregelmäßigen Haufen gelagert sind. Sie kommen physiologisch auf Haut und Schleimhäuten von Mensch und Tier vor. Im Prinzip lassen sich zwei Gruppen voneinander abgrenzen: der humanpathogene Staphylococcus aureus und der normalerweise zur physiologischen Flora gehörige S. epidermidis.

| S.epidermidis Gruppe | S.saprophyticus |
|---|---|
| - verschiedene Spezies | - Harnwegsinfektionen |
| - Hautflora | |
| - Infektionen von Plastik | |

Neben dem koagulaseproduzierenden S. aureus gibt es eine Reihe von Staphylokokkenspezies, die dieses Enzym nicht produzieren, deshalb Koagulase-negative Staphylokokken. Viele der zu dieser Gruppe gehörenden Bakterien besiedeln die Haut verschiedener Tiere. Wichtigster Vertreter beim Menschen ist S. epidermidis. Andere, beim Menschen normal vorkommende Staphylokokken sind S. hominis, S. haemolyticus und S. capitis. Krankheiten verursacht insbesondere S. epidermidis durch seine ausgeprägte Fähigkeit, Kunststoffmaterialien wie Katheter, künstliche Linsen, künstliche Herzklappen und andere Endoprothesen zu kolonisieren. Dabei bildet sich ein Biofilm auf der Oberfläche dieser Materialien, in denen die Bakterien nur schwer von Antibiotika und dem Immunsystem erreichbar sind.

Bei Akne, die gewöhnlich in Pubertätsalter auftritt, ist der anaerobe Mikroorganismus Propionibacterium acnes (P. acnes) maßgeblich an der Entstehung beteiligt und zersetzt das Sebum zu Glycerin und Fettsäuren, wodurch die Talgdrüsen wiederum angeregt werden vermehrt Sebum herzustellen und wobei diese Abbauprodukte die Follikelwandungen angreifen beziehungsweise zerstören. Dies ruft regelmäßig Entzündungen in der Haut (Pickel, Pusteln, Knoten, Zysten) hervor, welche oft nur narbig ausheilen, wodurch das optische Erscheinungsbild des an unreiner Haut leidenden Menschen dauerhaft geschädigt wird.

Herkömmliche Produkte zur Behandlung von fettiger und unreiner Haut, wie z.B. wässrigethanolische und/oder Tensid-haltige Reinigungsprodukte haben in der Regel den Nachteil die Haut zu strapazieren, auszutrocknen und wenig pflegend zu wirken. Besonders nachteilig ist, dass der Körper auf die Verwendung ethanolischer und/oder Detergentien-haltiger Lösungen über einen längeren Zeitraum hinweg mit einer Überproduktion von Sebum reagieren kann, was dem vorrangigen therapeutischen Ziel der Sebumreduktion bei fettig-öliger, unreiner und zu Akne neigender Haut genau entgegenwirkt.

Zur topischen Behandlung der Akne stehen Aknetherapeutika, wie zum Beispiel starke Oxidationsmittel, wie z.B Benzoylperoxid; alpha-Hydroxysäuren, wie z.B. Salicylsäure und Milchsäure; aliphatische Dicarbonsäuren, wie z.B. Azelainsäure; Retinoide, wie z.B. Tretionoin (Synonym: all-trans-Retinsäure), all-trans-Retinal und cis-13-Retinsäure (Isotretinoin); Antiandrogene (5alpha-Reductasehemmer) sowie Antibiotika, wie z.B. Clindamycin, Tetracyclin und Erythromycin zur Verfügung. Darüber hinaus kann eine abrasive Behandlung durchgeführt werden. Entsprechende Präparate enthalten Aluminiumoxid-Partikel oder Silikonharz.

Die Wirksamkeit der starken Oxidationsmittel, wie z.B. Benzoylperoxid; der alpha-Hydroxysäuren, wie z.B. Salicylsäure und Milchsäure sowie der aliphatischen Dicarbonsäuren, wie z.B. Azelainsäure wird dabei u.a. auf die Inhibition von P . acnes zurückgeführt. Die genannten Stoffe besitzen jedoch zumeist nur eine sehr moderate antimikrobielle Wirksamkeit gegenüber P. acnes und müssen daher in verhältnismäßig hoher Konzentration - Benzoylperoxid z.B. mit bis zu 5 Gew.-% und Azelainsäure mit bis zu 20 Gew.-% - in kosmetischen und dermatologischen Formulierungen eingesetzt werden. Bedingt durch die hohe Dosierung wird jedoch äußerst nachteilig die Haut sehr stark strapaziert, was sich insbesondere in starker Austrocknung der Haut äußert, die zum Teil aufgrund der starken Reduktion des pH-Wertes der Haut häufig mit starken Hautirritationen einhergeht.

Retinoide besitzen ein breites Wirksamkeitsspektrum. Die Wirkung beruht dabei insbesondere auf der Normalisierung der mit Akne einhergehenden follikulären Hyperkeratiose, der Auflösung von Komedonen und auf einer Inhibition der Sebumproduktion. Nachteilig ist jedoch, dass die meisten Retinoide ebenfalls stark irritierende Nebenwirkungen haben. Isotretinoin zeigt darüber hinaus eine teratogene Wirksamkeit und kann daher nicht zur Behandlung stark fettiger, unreiner und zu Akne neigender Haut von Schwangeren eingesetzt werden.

Die topisch sowie systemisch anzuwendenden Antibiotika, wie z.B. Clindamycin, Erythromycin und Tetracyclin besitzen eine stark antimikrobielle Wirksamkeit gegenüber P. acnes, wobei sie MHK-Werte im Bereich < 1 pmm aufweisen. Insbesondere aufgrund des in der klinischen Praxis immer häufiger zu beobachtenden Auftretens resistenter P. acnes Stämme sowie deren unspezifischem Wirkungsspektrum, welches zu einer starken Beeinträchtigung der gesunden humanen Mikroflora führt, geraten auch die in der Akne-Therapie geläufigen Antibiotika zunehmend ins Licht der Kritik.

EP 1269983 beschreibt 1,2-Decandiol als Mittel zur Hemmung des Wachstums von Körpergeruch verursachenden Keimen. Darin wird umfangreich zum Stand der Technik zu Diolen allgemein und zu Decandiolen im speziellen Bezug genommen.

EP 1915982 beschreibt die Verwendung von 1,2-Decandiol zur Reduktion der Sebumkonzentration der Haut und als Mittel zur Behandlung von Akne.

Zur Behandlung von Akne wird in EP 1598064 A1 eine Wirkstoffkombination aus alpha- und/oder beta-Hydroxysäuren und Alkan-1,2-Diolen vorgeschlagen, wobei beschrieben wird, dass die Kombination von 1 % Milchsäure mit 0,2 % 1,2-Decandiol eine verbesserte antibakterielle Wirksamkeit der Kombination in Bezug auf P. acnes gegenüber der Einzelbestandteile, insbesondere von 0,2 % 1,2-Decandiol aufweist. Bei der alleinigen Gabe von 0,2 % 1,2-Decandiol ist so gut wie keine antibakterielle Wirkung im Suspensionstest ersichtlich.

Alternativ oder kumulativ können bevorzugt entzündungshemmende bzw. rötungs-und/oder juckreizlindernde Wirkstoffe eingesetzt werden, die natürlich vorkommen. Geeignete Stoffe kommen in Pflanzenextrakten, speziell hochwirksamen Pflanzenextrakt-Fraktionen sowie aus Pflanzenextrakten isolierte hochreine Wirksubstanzen vor, und werden bevorzugt ausgewählt aus Kamille, Aloe vera, Commiphora-Arten, Rubia-Arten, Weiden, Weidenröschen, Hafer, Calendula, Arnika, Johanniskraut, Geißblatt, Rosmarin, Melisse, Ingwer, Passiflora incarnata, Hamamelis, Pueraria, Dianthus oder Echinacea sowie Reinsubstanzen, bevorzugt u.a. Bisabolol, Apigenin, Apigenin-7-glucosid, Rosmarinsäure, Boswelliasäure, Phytosterole, Glycyrrhizinsäure, Glabridin, Licochalkon A, Gingerole und Anthranilsäureamide, bevorzugt insbesondere Avenanthramid oder Dianthramid.

EP 1 938 785 A2 offenbart sebumregulierende Wirkstoffe, die ausgewählt sind aus Azelainsäure, Sebacinsäure, 10-Hydroxydecansäure, 1,10-Decandiol. Bevorzugte Dreierkombination wird in dem Handelsprodukt Acnacidol PG von Vincience eingesetzt.

WO 2008135085 A1 offenbart 1,2 Decandiol in synergistischer Kombination mit anderen antimikrobiellen Rohstoffen, wie Ethanol oder Triclosan.

Bekannt ist der Einsatz von Licochalconen in kosmetischen Zubereitungen. Licochalcone zeigen antimikrobielle Aktivität.

Nachteilig an den bekannten Akne-Zubereitungen ist, dass, um gegen Propionibacterium acnes (P. acnes) zu wirken, häufig der Anteil an antimikrobillen Wirkstoffen so hoch ist, dass die Zubereitungen hautirritierend sind.

Auch durch den Vorgang der Rasur kann es zu neuen Entzündungen der Haut kommen. Durch den Rasurvorgang moderner Apparate bzw. Mehrschichtklingen, werden die Haare tiefer abgeschnitten und liegen somit auch tiefer in der Haut. Somit kann es beim Herauswachsen zu eingewachsenen Bart-/Achselhaaren kommen. Diese können lokale Entzündungen der Haut hervorrufen. Gerade bei dunkelhäutigen Männer kann dies zu Hyperpigmentierungen, sogenannten Razor bumps (Pseudofolliculitis barbae) kommen. Aber auch bei Frauen kann es bei der Rasur im Bikinibereich zu solchen Entzündungen kommen. Ein einzelnes Produkt wird das Problem der Razor bumps nicht vollständig beseitigen können.
Bei der Follikulitis handelt es sich um eine Entzündung des oberen (äußeren) Anteils eines Haarbalgs, des Infundibulum. Meistens wird diese hervorgerufen durch das Bakterium Staphylococcus aureus, das sich massenhaft auf der menschlichen Haut befindet. Follikulitiden können an allen behaarten Körperstellen auftreten. Bevorzugte Stellen (Prädilektionsstelle) sind behaarte Kopfhaut, Brust- und Rumpfbereich. Besonders behaarte Männer sind in heißen Sommermonaten betroffen, wenn verstärktes Schwitzen das Bakterienwachstum zusätzlich fördert. Eine Follikulitis kann sehr schmerzhaft sein, die dabei entstandenen Papeln oder Pusteln heilen aber meistens folgenlos aus; der Haarbalg bleibt erhalten. Durch Verstopfung des Follikelausganges durch verquellendes Hornmaterial und besonders in Fällen verminderter Abwehrlage kann die Entzündung auf den gesamten Haarbalg übergreifen und damit zur Ausbildung eine Furunkels führen; ein Abszess entsteht durch die weitere Ausbreitung in das umliegende Gewebe. Beide müssen operativ saniert werden
Wünschenswert ist es eine kosmetische oder dermatologische Zubereitung zur Aknebehandlung bereit zu stellen, die eine verbesserte Wirkung gegen Propionibacterium acnes (P. acnes) aufweist und gleichzeitig weniger oder nicht Haut irritierend wirkt.

Ebenso wünschenswert ist es eine Zubereitung zur Verfügung zu stellen, die wenig Haut irritierend auch gegen Razur bumps einsetzbar ist.

Ebenso wünschenswert ist es eine Zubereitung zur Verfügung zu stellen, die wenig Haut irritierend auch gegen oder vorbeugend gegen Follikulitis einsetzbar ist.

Die Erfindung umfasst kosmetische oder dermatologische Zubereitungen umfassend die Kombination aus 1,2-Decandiol, Licochalcone A sowie Carnitin, wobei der Anteil an 1,2-Decandiol im Bereich von mehr als 0,1 Gew.%, bezogen auf die Gesamtmasse der Zubereitung, gewählt wird.

Durch die Kombination aus Licochalcone A, 1,2-Decandiol sowie Carnitin, lassen sich die P. acnes nachhaltig abtöten, ohne die Haut zu irritieren. Die erfindungsgemäßem Formulierung wirken Haut beruhigend und lassen die Zeichen der Akne schnell abklingen.
Neben der Verwendung als Aknemittel hat sich überraschenderweise auch der Einsatz der erfindungsgemäßen Kombination als Mittel gegen Entzündungen, die aufgrund der Rasur entstehen können, gezeigt.

Licochalcon A ist ein so genanntes Flavonoid, das aus der Wurzel des chinesischen Süßholzes (Glycyrrhiza inflata) extrahiert werden kann. Neben der bekannten antientzündlichen Wirkung konnten bisher antibakterielle, antiparasitäre und krebshemmende Wirkungen nachgewiesen werden. Licochalcone A hat die Struktur 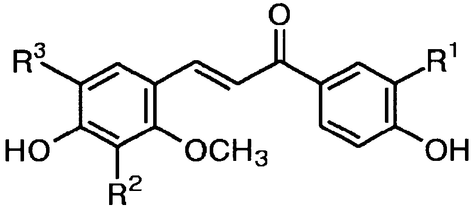 R¹ = H , R² = H , R³ = C(CH₃)₂-CH=CH₂ : Licochalkon A

Licochalcone A wird u.a. als Wirkstoff in Hautpflegeprodukten bei Neurodermitis und in Kosmetika für empfindliche Haut verwendet.

1,2-Decandiol umfasst erfindungsgemäß sowohl das 2S-konfigurierte Enantiomer als auch das 2R-konfigurierte Enantiomer sowie beliebige Mischungen aus 2S- und 2R-konfigurierten Decandiolen.
Aus kommerziellen Gründen ist es zwar besonders vorteilhaft, das Razemat des 1,2-Decandiols zur Behandlung von fettiger-öliger und unreiner Haut sowie zur Prophylaxe und/oder Behandlung von Seborrhoe, insbesondere Akne, einzusetzen, da diese synthetisch besonders leicht zugänglich sind, die reinen Enantiomere oder nicht-razemische Mischungen dieser Enantiomere sind aber ebenfalls für die erfindungsgemäßen Zwecke geeignet.

Carnitin, genauer L-Carnitin, ist eine natürlich vorkommende, vitaminähnliche Substanz. Es spielt eine essentielle Rolle im Energiestoffwechsel menschlicher, tierischer und pflanzlicher Zellen. Carnitin, auch {3-Hydroxy-4-(trimethylammonium)buttersäurebetain, [(R)-3-Carboxy-2-hydroxypropyl]trimethylammoniumbetain, oder Vitamin B_{T} bezeichnet, besitzt die Struktur 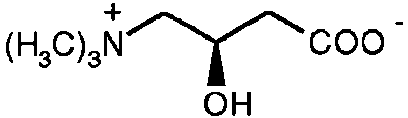 (3R)-, L-Form

L-Carnitin wird in zahlreichen Produkten zur Nahrungsergänzung angeboten. Zielgruppen sind (Ausdauer-)Sportler sowie übergewichtige Personen, denen L-Carnitin zur Leistungssteigerung bzw. als Schlankheitsmittel angeboten wird.

Alle drei erfindungsgemäßen Stoffe sind für sich dem Fachmann bekannt. So ist der Einsatz von 1,2 Decandiol zur Sebumreduktion angezeigt. Die Sebumreduktion reicht jedoch nicht aus, um Akne nachhaltig zu behandeln. Erfindungsgemäß wurde daher ermittelt, dass eine Aknebehandlung erst mit 1,2 Decandiol in Kombination mit dem endzündungshemmenden Wirkstoff Licochalcone A und dem Wirkstoff Carnitin, der Fette abbauen kann bzw. diese verflüssigt, nachhaltig und hautschonend erfolgen kann

Die antimikrobielle Wirksamkeit der erfindungsgemäßen Kombinationen gegen P. acnes wurde zum Vergleich mit verschiedenen Formulierungen untersucht.
Dazu wurde in einem Suspensionstest der Rückgang der P.acnes nach 1 h, 6h und 24h beobachtet. Dazu wurden wenige Einzelkolonien einer P.acnes - Kultur in 10mL anaerobem Medium im Impedanzgerät mit dem Programm PR37NT herangezogen. Nach der Wachstumsphase von ca. 18 Stunden wird zur Kontrolle der Keimzahl die OD gemessen, die etwa zwischen 0,3 - 0,5 liegen sollte. Für den Versuch wurden 100µL der gewachsene Kolonie und 100µL autoklaviertes Wasser zu 800mg der zu prüfenden Formulierung gegeben. Die Tests wurden manuell durchgeführt. Zwischen den Abnahmen werden die Proben unter anaeroben Bedingungen (Topf) gelagert. Die Proben wurden jeweils zum Zeitpunkt 1h, 6h und 24h genommen und auf den entsprechenden Agarplatten ausplattiert. Die Platten werden unter anaeroben Bedingungen (Topf) bei 37°C bebrütet. Nach 5 Tagen wurde die Keimzahl bestimmt.

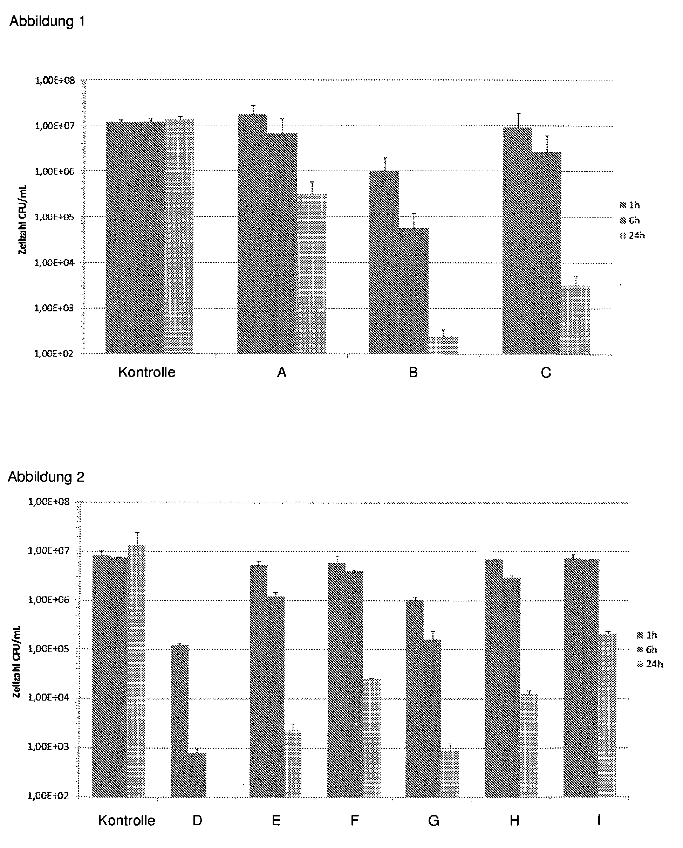

Die Abbildungen 1 und 2 zeigen die ermittelten Ergebnisse. Darin umfassen die Formulierung wie folgt
A: 0,5 Gew.% Licochalcone A
B: 0,5 Gew.% Licochalcone A und 0,3 Gew.% 1,2 Decandiol
C: 0,3 Gew.% 1,2 Decandiol
D: 0,5 Gew.% Licochalcone A, 0,3 Gew.% 1,2 Decandiol und 2 Gew.% Carnitin
E: 0,5 Gew.% Licochalcone A, 0,2 Gew.% 1,2 Decandiol und 2 Gew.% Carnitin
F: 0,5 Gew.% Licochalcone A, 0,1 Gew.% 1,2 Decandiol und 2 Gew.% Carnitin
G: 0,3 Gew.%-1,2 Decandiol und 2 Gew.% Carnitin
H: 0,2 Gew.% 1,2 Decandiol und 2 Gew.% Carnitin
I: 0,1 Gew.% 1,2 Decandiol und 2 Gew.% Carnitin

Die Formulierung B zeigt einen synergistischen Effekt der Kombination aus Licochalcone A und 1,2-Decandiol. 0,5% Licochalcone A allein zeigte keine antimikrobielle Wirksamkeit gegen P.acnes. Der Reduktionsfaktor betrug 41 nach 24h. 0,3% Decandiol allein zeigte nur eine schwache antimikrobielle Wirksamkeit. Die Zellzahl konnte um Faktor 4158 reduziert werden. Die Kombination Licochalcone A und 1,2-Decandiol zeigte jedoch eine antimikrobielle Wirksamkeit, die deutlich über der einfachen additiven Wirksamkeit der Einzelsubtanzen liegt. Der Reduktionsfaktor betrug nach 24h 55509.

Fügt man den Zubereitungen Carnitin hinzu, wird überraschenderweise der antimikrobielle Effekt gegen P.acnes nochmals verbessert, wie insbesondere die Zubereitung D zeigt. Reduziert man in den erfindungsgemäßen Formulierungen die Menge an Decandiol, so verringert sich auch die antimikrobielle Wirksamkeit. Damit ist der Anteil an 1,2 - Decandiol in den Zubereitungen vorteilhaft im Bereich von 0,2 Gew.% und mehr zu wählen.
Die Anteile an 1,2 - Decandiol werden im Bereich oberhalb von 0,1 Gew.%, insbesondere, wie die Untersuchungen zeigen, oberhalb von 0,2 Gew.% gewählt. Die Gewichtsanteile beziehen sich auf die jeweilige Gesamtmasse der Zubereitung.

Der Anteil an Llcochalcone A wird vorteilhaft im Bereich von 0,025 bis 1 Gew%, insbesondere im Bereich von 0,5 Gew.%, gewählt.
Als Licochalcone A Bestandteile kann erfindungsgemäß entsprechend auch Extrakte des Glycyrrhiza inflata verwendet und eingesetzt werden.

Carnitin wird bevorzugt zu einem Anteil von 1 Gew.% bis zu 5 Gew.% den Zubereitung zugesetzt.

Die erfindungsgemäßen Zubereitungen können als kosmetische oder dermatologische Zubereitung in Form von Emulsionen, hier insbesondere W/O-, O/W- oder W/O/W-Emulsionen, als Gele, Hydrodispersionen, Toner, Scrub, zwei- oder mehrphasige Systeme, Lichtschutzzubereitungen, mattierende Puder, Seren, Stick, Suspensionen, als

Tränkungsmedien auf Tücher oder Pads als auch in transdermalen Matrices eingesetzt und angeboten werden.

In Form von Mousse oder Schaum ergeben sich weitere vorteilhafte Applikationsformen. Bevorzugt sind O/W- oder W/O-Pflegeemulsionen umfassend die Kombination aus 1,2-Decandiol, Licochalcone A und vorteilhaft Carnitin.
Die erfindungsgemäßen Zubereitungen können in üblichen Packungsvarianten, wie insbesondere als Aerosol, Pumpspray, Tube oder im Tiegel angeboten werden.

Vorteilhaft für den sichtbaren Erfolg derartiger Produkte ist die Anwendung in einer bestimmten Folge. Diese Art der Benutzung wird üblicherweise als Systempflege bezeichnet und spiegelt die Benutzung der auf einander abgestimmten Produkte wieder.
Diese Systempflege besteht im Wesentlichen aus 5 Schritten:

### 1. Reinigung

Bei unreiner Haut spielt die Reinigung eine besondere Rolle für ein sichtbar verbessertes Hautbild. Tägliches Reinigen reduziert Talgüberschuss und Bakterien. Ein Produkt mit Ampho-Tensid reinigt sanft und intensiv und befreit die Haut von überschüssigem Talg. Morgens und abends auf die Fingerspitzen geben und sanft auf das Gesicht auftragen. Anschließend mit lauwarmem Wasser abspülen.

### 2. Peeling

Zubereitungen mit Peelingkörper öffnen die Poren und reduzieren Unreinheiten und Mitesser. So wird das Hautbild verfeinert. Eine kleine Menge des Peelings in den Händen aufschäumen und ein bis zwei Minuten sanft einmassieren. Anschließend mit lauwarmem Wasser abspülen. Je nach Empfindlichkeit der Haut kann das Peeling 1- bis 2-mal pro Woche, bei Bedarf auch täglich, angewendet werden.

### 3. Tonisieren

Häufig wird das Tonic bei der täglichen Gesichtspflege vergessen. Für unreine Haut ist es aber sehr wichtig, denn es klärt die Haut porentief und hat eine komedolytische (Poren öffnende) Wirkung. Zubereitungen mit alpha-Hydroxysäuren (z.B. Milchsäure) öffnen verstopfte Poren und klärt und erfrischt die Haut gezielt. Am besten mit einem Wattepad auftragen - Wattepads aber nicht mehrmals verwenden!

### 4. Pflege

Die richtige tägliche Pflege kann die gestörten Hautfunktionen gezielt regulieren. Bisher waren ölfreie Pflegeprodukte für unreine Haut ideal, weil sie nicht zusätzlich der Pickelbildung Vorschub leisten, indem sie die Haut mit Lipiden abdichten. Die erfindungsgemäße Zubereitung mit Decandiol, Licochalcon A und Carnitin reduziert wirksam Hautunreinheiten und beugt der Entstehung neuer vor. Diese Art der Zubereitungen müssen nun nicht mehr ölfrei sein und vermindern trotzdem die sichtbaren Zeichen der Akne. Täglich morgens und abends aufgetragen, verbessert es das Hautbild innerhalb von kurzer Zeit sichtbar: Der Teint strahlt wieder frischer, gleichmäßiger und klarer.
Mit dem Wirkkomplex aus Decandiol, Licochalcon A und Carnitin können Menschen mit Akne nun auch mattierende und UV-Filterhaltige Produkte und sogar Nachtcremes nutzen. Eine ölfreie oder lipidarme Formulierung ist nun nicht mehr notwendig.

### 5. Kaschieren

Auf das selbst ausdrücken von Pickel soll aufgrund der Narbenbildung verzichtet werden. Damit Hautunreinheiten und störende Pickel schneller abklingen, sollte ein Abdeckstift 1-3 täglich direkt auf die betroffenen Stellen auftragen. Der praktische Abdeckstift ist (auch unterwegs) hygienisch anzuwenden, reduziert die Unreinheiten nachweisbar und kaschiert sie farblich zuverlässig.

Auch zur Vermeidung von eingewachsenen Haaren ist eine Systempflege notwendig. Die Kombination von mindestens zwei, idealerweise drei, speziellen Produkte kann auch dieses Problem gelöst werden.
1. Ein mechanisches Peeling, welches einer Verhornung der Epidermis entgegenwirkt und so ein Einwachsen der Barthaare erschwert.
2. Ein Rasiermittel, welches einen ausgeprägten Gleitfilm auf der Haut bildet, so dass die Barthaare oberhalb der Hautoberfläche abgeschnitten werden und ggf.
3. ein leichtes Pflegeprodukt mit bakteriziden und Anti-Irritativen Wirkstoffen, welche eine Hyperpigmentierung verhindern, wie es erfindungsgemäß durch die Kombination aus Decandiol, Licochalcon A und Carnitin angeboten wird.

Vorteilhaft kann die erfindungsgemäße Kombination nicht nur in bekannten Hautpflegeprodukten verwendet werden, sondern in allen zur Behandlung von Akne oder zur Vermeidung von Entzündungen geeigneten Zubereitungsformen.
So lassen sich die Decandiole, Liccochalcone A und Carnitin sowohl in Reinigungs-, Peeling-, make-up-Zubereitungen als auch in Rasierzubereitungen einsetzen, wie auch die Beispielzubereitungen darlegen.
Es ist damit ein System an Hautbehandlung und -pflege möglich, indem die einzelnen Systemkomponenten, bevorzugt alle, die erfindungsgemäße Kombination umfassen.

Bevorzugte Systemkomponenten sind Zubereitungen zur Reinigung, Peeling, Tonisierung, Pflege und/oder zum Kaschieren.
Als Peelingzubereitung unmfasst die Zubereitung vorteilhaft 0,1 bis 5 Gew.% Peelingkörper, die aus bekannten Körpern, wie Polyethylen, Kochsalz, Meersalz, Natriumcarbonat, Natriumhydrogencarbonat, Magnesiumsulfat, Magnesiumchlorid, Zucker, Tonerde, Sand, Kunststoffpartikel, zerstoßene oder zermahlene Kerne von Walnusschalen, Aprikosen-, Pfirsich- oder Mandelkernen gewählt werden können.

Als Rasurzubereitungen oder zur Vorbeugung von Follikulitis werden die erfindungsgemäßen Zubereitungen vorteilhaft unmittelbar nach der Rasur, zur Entkeimung und zur Vorbeugung von einwachsenden Haaren als Systemkomponenten angeboten bzw. verwendet.
1,2 Decandiol, Licochalcone A und Carnitin kann zur Behandlung der Haut unmittelbar nach der Rasur, zur Entkeimung und/oder zur Vorbeugung von einwachsenden Haaren verwendet werden.

Die Erfindung besteht daher auch aus einem kosmetischen System zur Hautpflege bzw. - behandlung umfassend mindestens zwei Zubereitungen, die jeweils die erfindungsgemäße Kombination umfassen.
Weiterhin kann das System, umfassend mindestens zwei, vorteilhaft 3 bis 5 Zubereitungen, räumlich zueinander angeordnet sein, wie z.B. nebeneinander in einem Verkaufsregal oder durch eine Kombinationspackung, die zwei oder mehrere der Zubereitungen umfasst. Der Anwender erhält somit ein aufeinander abgestimmtes Kosmetikkonzept ohne in verschiedenen Läden, Abteilungen, Regalen die zueinander passsenden Mittel zu suchen. Ebenso ist es erfindungsgemäß das System so aufeinander abzustimmen, dass die einzelnen Zubereitungen zeitlich aufeinander abgestimmt, z.B. in bestimmter Reihenfolge, angewendet werden können.

Die kosmetischen und dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften hinsichtlich der Akne-, Follikulitis- oder razor bumps-Behandlungen nicht beeinträchtigt.

Den erfindungsgemäßen Zubereitungen sind vorteilhaft Feuchthaltemittel bzw. sogenannte Moisturizer zugesetzt.
Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Bevorzugt liegt der Anteil an Hautbefeuchtungsmitteln, auch Moisturizer genannt, im Bereich von 1 bis 60 Gew%, besonders bevorzugt von 1 bis 35 Gew% und ganz besonders bevorzugt von 1-25 Gew%.
Vorteilhafte Moisturizer im Sinne der vorliegenden Erfindung sind insbesondere Glycerin und/oder Milchsäure. Weiterhin sind auch Butylenglykol, Sorbitol, Pyrrolidoncarbonsäure, polymere Moisturizer aus der Gruppe der wasserlöslichen und/oder in Wasser quellbaren und/oder mit Hilfe von Wasser gelierbaren Polysaccharide als Moisturizer zu verwenden. Insbesondere vorteilhaft sind beispielsweise kurzkettige Hyaluronsäure (< 50.000 Dalton) und langkettige Hyaluronsäure (> 50.000 Dalton), Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.
Milchsäure ist bevorzugt zu einem Anteil von 1 bis 5 Gew.% in der Zubereitung enthalten. Vorteilhaft sind den erfindungsgemäßen Zubereitungen auch Parfuminhaltsstoffe zugesetzt, wobei sich deren antimikrobielle Wirksamkeit weiter steigern lässt und zugleich ein angenehmeres olfaktorisches Befinden erzielen lässt.
Vorteilhaft sind insbesondere die Parfuminhaltsstoffe gewählt aus: (1-oxopropoxy)-,1-(3,3-dimethylcyclohexyl), (süßes) Majoranöl, 1,1-Dimethoxy-2,25-trimethyl-4-hexene, 1,2 Hexandiol, 1,2-Propylene glycol, 1,3,4,6,7,8-Hexahydro-4,6,6,7,8,8-hexamethylcyclopenta-gamma-2-benzopyran, 1,4-Dioxacyclohexadecane-5,16-dione, 1,5,9-Trimethyl-13-oxabicyclo(10.1.0) trideca-4,8-diene, 1,6,7,8-Tetrahydro-1,4,6,6,8,8-hexamthyl-as-indacen-3<2H>-one, 12-Oxahexadecanolide, 1-Phenyl-3-methyl-3-pentanol, 2,4-Dimethyl-3-cyclohexen-1-carboxaaldehyde, 2,6-Dimethyl-7-octen-2-ol, 2,6-Nonadienal, 2-Acetonapthone-1,2,3,4,5,6,7,8-octahydro-2,3,8,8-tetramethyl, 2-Isobutyl-4-hydroxy-4-methyltetrahydropyran, 2-Methyl-3(4-(2-methylpropyl)phenyl)propanal, 2-Methyl-4-(2,6,6-trimethyl-2(1)-cyclohexen-1-yl) butanal, 2-Methyl-4-phenyl-2-butanol, 2-Methyl-4-phenyl-2-pentanone, 2-Methyl-5-phenylpentan-1-ol, 2-methyl-6-methylene, 2-Methylbutyl acetate, 2-Phenoxyethyl isobutyrate, 2-tert-Pentyl-cyclohexyl acetate, 3(4),8(9)-Dihydroxymethyl tricyclo(5.2.1.0(2,6)decane, 3-(4-t-Butylphenyl)propanal, 3-(5,5,6-Trimethylbicyclo(2.2.1)hept-2-yl)cyclohexan-1-ol, 3,7,11-Trimethyl-1,2,10,-dodecatrien-3-ol (cis & trans), 3,7-Dimethyl-1,3,6-octatriene, 3,7-Dimethyl-1-octanol, 3,7-Dimethyl-2(3),6-nonadienenitrile, 3,7-Dimethyl-2,6-octadien-1-ol, 3,7-Dimethyl-6-octen-1-ol, 3,7-Dimethyloctan-3-yl acetate, 3-Isocamphyl cyclohexanol, 3-Methyl-5-phenyl-1-pentanol, 3-Pentyltetrahydro(2H)pyranyl acetate, 3-Phenyl-1-propanol, 4-(4-Hydroxy-4-methylpentyl)-3-cyclohexene-1-carboxyaldehyde, 4-(p-Hydroxyphenyl)-2-butanone, 4-Acetyl-6-t-butyl-1,1-dimetylindane, 4-Carvomenthenol, 4-t-Butylcyclohexyl acetate, 5-Phenylpentanol, 7-Acetyl-1,1,3,4,4,6-hexamethyltetralin, 7-Octen-2-ol, Abietyl acetate, Acetic acid, Acetyl Hexamethyl Tetralin (Moschus-Verb.), Acetyl Trifluoromethylphenyl Valylglycine, Acrylamidammoniumacrylatcopolymer, Allyl phenoxyacetate, alpha-Amylcinnamaldehyde, alpha-Butylcinnamaldehyde, alpha-Hexylcinnamaldehyde, alpha-lonone, alpha-Isomethylionone, alpha-Methyionantheme, alpha-Methyl-3,4-methylene dioxyhydrocinnamic aldehyde, alpha-Methylbenzyl acetate, alpha-Pinene, alpha-Terpineol, Aluminummagnesiumhydroxidstearat, Amaranthus Extract, Ammoniumlactat, Ammoniumpolyacrylat, Ammoniumpolyacryloyldimethyltaurat, Amylacetat, Amylsalicylat, Andrographolide, Anethol, Anisic acid, Anisöl, Anisyl acetate, Anisyl alcohol, Annatto, Anthemis Nobilis Flower, Arginin PCA, Ascorbyl Tocopheryl Maleate, Avena Aqua, Bayöl, Benzaldehyde, Benzyl acetate, Benzyl alcohol, Benzyl benzoate, Benzyl Cinnamate, Benzyl laurate, Benzyl salicylate, Bergamot oil, Bergamot oil, bergaptene free, beta-Caryophyllene, beta-Ionone, beta-Pinene, BGT, Biosaccharide Gum 3, Bois de rose oil, Bornyl acetate, Butyl Hydroquinone, Butylated hydroxytoluene, Calendulaöl, Camphene, Capryloylsalicylsäureester, Carnaubau Wax, Carvon , Cassia Alata, Cedarwood oil, Cedrenol, Cedrenyl acetate, Cedrol, Cedrol methyl ether, Cedryl acetate, Chlorhexidine Digluconate, Chrysanthenum Parthenium, Cinnamic alcohol, Cinnamic aldehyde, cis-3-Hexenyl salicylate, Citral, Citronella oil, Citronellal, Citronellyl acetate, Citrus Aurantium Dulcis Blossoms, Citrus oil distilled, Clove bud oil, Cocoglucosid, Coffea Robusta, Cognac oil, Coleus Barbatus Extract, Coumarin, Cyclamen aldehyde, Cyclohexyl salicylate, d-Camphor, Decanal, delta-3-Caren, Diacetin, Diethyl malonate, Diethyl phthalate, Diethylene glycol monoethyl ether, Dihydromyrcenyl acetate, Dihydroterpinyl acetate, Dimethoxy Di-p-Cresol, Dimethyl benzyl carbinyl acetate, Dimethyl benzyl carbinyl butyrate, Dimethyl phthalate, Dimethylbrassylat, Dinatriumadenosintriphosphat, Dipenten , Diphenyl ether, Diphenyldimethicon, Dipropylene glycol, Disodium NADH, Disteardimoniumhectorit, d-Limonene, Natriumsuccinat, Dodecene, Echium Lycopsis Oil, Eclipta Prostrata Extract, Eperua Falcata , Erythritol, Essential Oils, Ethyl acetate, Ethyl acetoacetate, Ethyl Bisiminomethylguaiacol Manganese Chloride, Ethyl butyrate, Ethyl Glucoside, Ethyl maltol, Ethyl vanillin, Ethyl-2methyl-1,3-dioxolane-2-acetate, Ethylene brassylate, Ethyllinalool, Ethyllinylyl acetate, Ethylvanillin, Etyhl methylphenylglycidate, Eucalyptol, Eucalyptus oil, Eugenol , Extrakt aus Blättern der Melisse, Fir needle oil, Formaldehyde cyclododecyl ethyl acetal, Galbanum oil, gamma-Decalactone, gamma-Nonalactone, gamma-Terpinene, gamma-Undecalactone, Gartenrautenöl, Geranium oil, Geranyl acetate, Glucosylrutin, Glyceryl caprylate, Grapefruit oil, Guanosine, Gum Benzoin, Heliotropin, Heptapeptide-6, Hexahydro-4,7-methanoinden-5(6)-yl acetate, Hexyl acetate, Hexyl alcohol, Hexyl salicylate, Hexylene glycol, Hierochloe Odorata Extract, Holunderextrakt, Holunderöl, Hopfenöl, Humulus Japonicus Flower / Leaf / Stem Extract, Hydrated Magnesium Silicate, Hydrierte Palmenkernglyceride, hydriertes Palmenglycerid, hydriertes Palmenglyceridcitrat, Hydrogenated Myristyl Olive Esters, Hydrolisiertes Weizenprotein PG-Propylmethylsilandiol, Hydrolyzed Cera Alba, Hydrolyzed Wheat Protein PG-Propyl Methylsilanediol, Hydroxycitronellal, Hydroxycitronellol, Hydroxyethylacrylat / Natriumacryloyldimethyltauracopolymer, Hydroxyethylpiperazine Ethane Sulfonic Acid, Hydroxypropyl Tetrahydropyrantriol, Ingweröl, lonone, Isoamyl salicylate, Isoamylacetat, Isobornyl acetate, Isobornylcyclohexanol, Isobutyl salicylate, Isodeceth-6, Isoeugenol, Isopropyl myristate, Isoquercitrin, Juniper berry oil, Kaempferia Galanga Root Extract, Kamelienöl, Kamillenöl, Kampher, Kardamomöl, Kiefern(teer)öl, Kiefernöl, Kreuzkümmelextrakt, Kümmelöl, Kupfergluconat, Labdanum oil, Lagerstroemia Indica Extract, Lauric aldehyde, Lauryl alcohol, Lauryl Dimonium Hydroxypropyl Hydrolyzed Soy Protein, Lauryl PolyglucosideSodium Hydroxypropyl Starch Phosphate, Lavandin oil, Lavender oil, Lemon oil, Lemon oil terpenes, Lemon terpenes, Lemongrassöl, Liebstöckelöl, Lime oil, Linalool, Linalyl acetate, Linseed Acid, Linseed Acid Magnesiumaspartat, I-Menthol, Lorbeeröl, Lupinus Luteus Extract, Majantol, Maltol isobutyrate, Mandarin oil, Melibiose, Menthanyl acetate, Menthol, Menthol liquid, Menthone, Menthyl acetate, Menthyllactat, Menthylsalicylat, Methyl 2-nonenoate, Methyl abietate, Methyl cedryl ketone, Methyl dihydrojasmonate, Methyl ester of rosin, Methyl Rosinate, Methyl-alpha-ionone, Methyl-deltaionone, Methyldihydrojasmonate, Methyleugenol, Methylpentenolone, Methylserine, Methylsilanol / Silicate CrosspolymerAmaranthus Caudatus Extract, Mineral oil, Musk ketone, Muskatnussöl, Myrcene, Myrcenyl acetate, Myristicin, Natriummetabisulfit, Nelkenöl, Nelkenblattöl, Nerol, Neroli bigarade oil, Nerolidol, Neryl acetate, Nicotinamide, Nopyl acetate, Nutmeg oil, Nylon-66, Ocotea Cymbarum Öl, Octanal, Octyl acetate, Octyldodecanol, Öl aus Blättern der Melisse, Oleyl Erucate, omega-Pentadecalactone, Orange oil, Orange terpenes, Orange terpenes (natural), Orangenblütenöl, Orangenblütenwasser, Orangenextrakt, Orangenschalenextrakt, o-t-Butylcyclohexyl acetate, Oxacyclohexadecen-2-one, Oxothiazolidinecarboxylic Acid, Oxothiazolidinecarboxylsäure, Palmitoyl Lysyl Aminovaleroyl Lysine, Palmitoyl Tetrapeptide-10, Palmitoylpentapeptide 4, Patchouli oil, p-Cymene, PEG / PPG-14/7 Dimethyl Ether, PEG-10 Dimethicone / Vinyl Dimethicone Crosspolymer, PEG-2 Stearyl Ether, PEG-20 Stearyl Ether, PEG-24 Cetyl Ether, PEG-24 Cholesteryl Ether, PEG-60 Glyceryl Isostearate, PEG-8 Laurat, Penethyl Alcohol, Pentadecalacton, Penthylene Glycol, Peppermint oil, Petersilienöl, Petitgrain oil, Pfefferminzextrakt, Pfefferminzöl, Phenethyl alcohol, Phenoxyethanol, Phenylacetaldehyde glyceryl acetal, Phenylethyl acetate, Phenylpropanol, Phytol, Pisces Collagen, Platanus Occidentalis, p-Methylanisole, Poly C10-30 Alkyl Acrylate, Polyacrylate-13, Polyacrylate-3 , Polycaprolactone, Polyester-5, Polyglyceryl-2 Caprate, Polyglyceryl-6 Polyricinoleate zugesetzt sein., p-t-Butyl-alpha-methyldihydrocinnamic aldehyde, Pulegone, Punica Granatum Fruit Juice, Pyracantha Fortuneana Extract, Pyrus Malus Water, Rhodinol, Rosemary oil, Rosenextrakt, Rosenöl, Rosmarinöl, Sabinene, Salbeiöl, Sandalwood oil, Sandelholzöl, Sarcosin, Sassafrasbaumöl , Saxifraga Sarmentosa Extrakt, Schafgarbenöl, Sclareoate, Scutellaria Baicalensis Extrakt, Selaginella Tamariscina Extract, Shorea Robusta Butter, Sodium Dodecylbenzene Sulfonate, Sodium Hydroxyethyl Acrylate / Acryloyldimethyl Taurate Copolymer, Sodium Mannose Phosphate, Sojaisoflavon, Spearmint oil, Spike lavender oil, Stearic acid, Succinoglycan, Sucrose Polycottonseedate, Teebaumöl, Teeröl, Terpineol, Terpineol, Terpinyl acetate, Tetrahydrobisdemethoxycurcumin, Tetrahydrobisdemethoxydiferuloylmethane, Tetrahydrodemethoxycurcumin, Tetrahydrodemethoxydiferuloylmethane, Tetrahydrolinalool, Tetrahydromyrcenol, Thiodipropionic Acid, Thiotaurine, Thyme oil, Thymianöl, Thymol, Tocopheryl Glucoside, Tocopherylglucosid, trans-Anethole, trans-beta-Ionone, Treemoss abs., Tri C14-15 Alkylcitrate, Triacetin, Trichloromethyl phenyl carbinyl acetate, Tricyclodecenyl propionate, Trideceth-6, Triethyl citrate, Trioctyldodecyl Citrate, Trioxaundecanedioic Acid, Undecanal,2-methyl-, Vanille, Vanillin, Vetiver oil, Wacholderteer, Weihrauchharz, Weihrauchharzextrakt, Xymenynic Acid, Ylang ylang oil, Zimtöl, Zimtsäure, Zinkgluconat , Zitronengrasöl, Zitronenöl,

Die erfindungsgemäßen Zubereitungen sind topisch applizierbar formuliert. Unter topisch applizierbaren Zubereitungen werden erfindungsgemäß Lotionen, Gele, Emulsionen, Aerosol-Mousse, pumpbare Zubereitungen, Cremes sowie damit getränkte oder beladene Tücher, Pads oder Pflaster verstanden.

Die nachfolgenden Beispiele zeigen kosmetische Zubereitungen mit uneingeschränkter Wirkleistung gegen Propionibacterium acnes (P. acnes). Die Zahlenangaben sind Gewichtsanteile bezogen auf die Gesamtmasse der jeweiligen Zubereitung.

### Beispiele

**Reinigungsgel**

| | 1 | 2 | 3 | 4 | 5 |
|---|---|---|---|---|---|
| Propylenglycol | 5,0 | 5,0 | 7,5 | 5,0 | 15,0 |
| Wasser | ad 100 | ad 100 | ad 100 | ad 100 | ad 100 |
| Zitronensäure | 1,2 | 1,2 | 1,2 | 1,2 | 1,2 |
| Natriumbenzoat | 0,25 | 0,25 | 0,25 | 0,25 | 0,25 |
| Natriumlaurethsulfat | 4,3 | 4,8 | 4,5 | 4,3 | 8,3 |
| Licochalcone A | 0,35 | 0,25 | 0,55 | 0,05 | 1,35 |
| Carnitin | 1,0 | 2,0 | 1,0 | 5,0 | 1,35 |
| Decandiol | 0,1 | 0,15 | 0,55 | 1,0 | 1,35 |
| Salicylsäure | 0,1 | 0,1 | 0,1 | 0,1 | 0,1 |
| Natriumcocoamphoacetat | 19,5 | 21,5 | 19,5 | 9,5 | 19,5 |
| Natriumchlorid | 0,01 | 0,01 | 0,014 | 0,01 | 0,1 |

**Peeling-Mittel**

| | 6 | 7 | 8 |
|---|---|---|---|
| Methylparaben | 0,35 | 0,35 | 0,15 |
| Propylparaben | 0,35 | 0,15 | 0,35 |
| Wasser | ad 100 | ad 100 | ad 100 |
| Phenoxyethanol | 0,95 | 0,65 | 0,55 |
| Benzophenone-4 | 0,05 | 0,05 | 0,05 |
| PEG-40 hydriertes Rizinusöl | 0,5 | 0,5 | 0,75 |
| Natriummyrethsulfat | 2,9 | 2,9 | 2,9 |
| Polyquaternium-10 | 0,1 | 0,1 | 0,1 |
| Polyethylen-Scrub fein | 1,7 | 1,7 | 2,0 |
| Cocamidopropylbetaine + Glycerin | 11,8 | 11,75 | 15,0 |
| Decyl Glucoside | 4,0 | 4,0 | 4,5 |
| Licochalcone A | 0,15 | 0,15 | 0,15 |
| Carnitin | 2,0 | 3,0 | 1,0 |
| Decandiol | 0,25 | 0,2 | 1,0 |
| Polyethylene-Scrub mittelgrob | 0,30 | 0,30 | 0,50 |
| Acrylates/C10-30 Alkylacrylatcrosspolymer | 0,75 | 0,75 | 0,75 |
| PEG-90 Glyceryl Isostearate + Laureth-2 | 0,15 | 0,15 | 0,15 |
| Hydroxypropylstärkephosphat | 1,0 | 1,0 | 1,0 |
| Parfum | 0,25 | 0,25 | 0,35 |
| farbige Partikel aus Mikrokristalline Cellulose + Cl 77289 als Zeitindikator | 0,3 | 0,3 | 0,3 |

**Tagespflege mit Lichtschutzfilter**

| | 9 | 10 | 11 | 12 |
|---|---|---|---|---|
| Methylparaben | 0,3 | 0,3 | 0,3 | 0,3 |
| Wasser | ad 100 | ad 100 | ad 100 | ad 100 |
| Cetearylalkohol | 3,5 | 5,5 | 3,5 | 3,5 |
| Natrium-EDTA | 1,0 | 1,0 | 1,0 | 1,0 |
| Phenoxyethanol | 0,6 | 0,6 | 0,6 | 0,6 |
| lineares Silikonöl | 1,0 | 1,0 | 1,0 | 1,0 |
| zyklisches Silikonöl | 1,0 | 2,0 | 3,0 | 1,0 |
| C12-15 Alkylbenzoat | 5,0 | 7,5 | 5,0 | 5,0 |
| Butylmethoxydibenzoylmethan | 4,5 | 5,5 | 4,5 | 4,5 |
| Phenylbenzimidazolsulfonsäure, Natriumsalz | 3,5 | 5,5 | 3,5 | 3,5 |
| Glycerin | 6,0 | 6,0 | 6,0 | 6,0 |
| Acrylates/C10-30 Alkylacrylatcrosspolymer | 0,2 | 0,4 | 0,2 | 0,2 |
| Octocrylene | 4,5 | 7,5 | 4,5 | 4,5 |
| Chondrus Crispus | 0,25 | 0,55 | 0,25 | 0,25 |
| Bis-Ethylhexyloxyphenolmethoxyphenyl Triazin | 3,0 | 3,0 | 3,0 | 3,0 |
| Tapiokastärke | 4,0 | 5,0 | 4,0 | 4,0 |
| Carnitin | 2,0 | 2,0 | 2,0 | 1,0 |
| Licochalcone A | 0,35 | 0,5 | 0,35 | 0,35 |
| Decandiol | 0,3 | 0,5 | 0,35 | 0,3 |
| Natriumstearoylgtutamat | 0,2 | 0,5 | 0,4 | 0,2 |
| Glycerylglucosid | 6,0 | 6,0 | 16,0 | 6,0 |

**mattierende Tagespflege**

| | 13 | 14 | 15 | 16 |
|---|---|---|---|---|
| Wasser | ad 100 | ad 100 | ad 100 | ad 100 |
| Octyldodecanol | 2,0 | 2,2 | 4,0 | 2,0 |
| Cetearylalkohol | 3,2 | 3,0 | 3,5 | 2,0 |
| Phenoxyethanol | 0,6 | 0,6 | 0,6 | 0,6 |
| lineares Silikonöl | 1,0 | 1,0 | 1,0 | 1,0 |
| Glycerin | 6,0 | 6,0 | 6,0 | 6,0 |
| Butylenglykol | 1,0 | - | 1,0 | 1,0 |
| Ethanol | 4,0 | 4,0 | 8,0 | 4,0 |
| Acrylates/C10-30 Alkyl Acrylatcrosspolymer | 0,2 | 0,2 | 0,3 | 0,2 |
| Tapiokastärke | 4,0 | 2,0 | 8,0 | 4,0 |
| Carnitin | 2,0 | 2,0 | 5,0 | 2,0 |
| Licochalcone A | 0,035 | 0,035 | 0,35 | 0,035 |
| Decandiol | 0,3 | 0,6 | 1,2 | 0,3 |
| Natriumstearoylglutamat | 0,2 | 0,2 | 0,6 | 0,2 |

**Aktivkonzentrat**

| | 17 | 18 | 19 | 20 |
|---|---|---|---|---|
| Wasser | ad 100 | ad 100 | ad 100 | ad 100 |
| Phenoxyethanol | 0,6 | 0,6 | 0,6 | 0,6 |
| zyklisches Silikonöl | 10,0 | 10,0 | 8,0 | 10,0 |
| Milchsäure | 2,0 | 2,0 | 1,0 | 2,0 |
| Glycerin | 10,0 | 10,0 | 20,0 | 10,0 |
| Xanthan Gummi | 0,4 | 0,4 | 0,8 | 0,4 |
| Tapiokastärke | 4,0 | 4,0 | 4,0 | 4,0 |
| zyklisches Silikonöl + Dimethiconol | 5,0 | 5,0 | 5,0 | 5,0 |
| Methylpropanediol | 2,0 | 2,0 | 4,0 | 2,0 |
| Carnitin | 1,0 | 1,0 | 2,5 | 1,0 |
| Licochalcone A | 0,25 | 0,25 | 0,25 | 0,5 |
| Decandiol | 0,3 | 0,35 | 0,6 | 0,25 |
| Parfum | 0,75 | 0,75 | 0,5 | 0,35 |
| Natriumstearoylglutamat | 0,22 | 0,2 | 0,4 | 0,6 |

**Nachtpflege**

| | 21 | 22 | 23 | 24 | 25 |
|---|---|---|---|---|---|
| Methylparaben | 0,1 | 0,1 | 0,1 | 0,1 | 0,1 |
| Wasser | ad 100 | ad 100 | ad 100 | ad 100 | ad 100 |
| Bisabolol | 0,1 | 0,1618 | 0,1 | 0,1 | 0,1 |
| Cetearylalkohol | 2,0 | 3,0 | 2,0 | 2,0 | 2,0 |
| Sorbitanstearat | 2,0 | 3,0 | 2,0 | 2,0 | 2,0 |
| Natrium- EDTA | 1,0 | 1,0 | 1,0 | 1,0 | 1,0 |
| Phenoxyethanol | 0,3 | 0,35 | 0,37 | 0,3 | 0,6 |
| zyklisches Silikonöl | 5,0 | 5,0 | 5,0 | 5,0 | 5,0 |
| Panthenol | 1,0 | 1,618 | 2,5 | 10,0 | 1,4 |
| Milchsäure | 2,0 | 2,5 | 1,0 | 2,0 | 2,0 |
| Carnitin | 3,0 | 1,618 | 3,0 | 3,0 | 3,0 |
| Licochalcone A | 0,125 | 0,1618 | 0,125 | 0,125 | 0,125 |
| Decandiol | 1,618 | 1,618 | 1,618 | 1,618 | 1,618 |
| Glycerin | 5,0 | 5,0 | 5,0 | 5,0 | 5,0 |
| Xanthan Gummi | 0,5 | 0,75 | 0,5 | 0,5 | 0,5 |
| Serin + Lecithin + Glycin + Alanin | 0,5 | 0,5 | 0,5 | 0,5 | 0,5 |
| Polyglyceryl-3 Methylglucosedistearat | 5,0 | 7,5 | 5,0 | 5,0 | 5,0 |
| Cl 77891 | 0,1 | 0,1 | 0,1 | 0,1 | 0,1 |
| PEG-150 Distearat | 1,0 | 1,6 | 1,0 | 1,9 | 1,0 |
| Parfum | 0,15 | 0,15 | 0,15 | 0,15 | 0,15 |

**Rasierschaum**

| | 26 | 27 | 28 | 29 | 30 |
|---|---|---|---|---|---|
| Stearinsäure | 5.5000 | 5.5000 | 7.5000 | 5.5000 | 5.5000 |
| Propylenglykol | 1.0000 | 1.0000 | 3.0000 | 1.0000 | 1.0000 |
| Wasser | ad 100 | ad 100 | ad 100 | ad 100 | ad 100 |
| Bisabolol | 0,2 | 0,2 | 0,5 | 0,2 | 0,35 |
| Jojobaöl | 0,1 | 0,1 | 0,1 | 0,1 | 0,1 |
| Laureth-23 | 2,75 | 2,9 | 2,75 | 2375 | 2,75 |
| Nylon-12 | 0,5 | 0,5 | 0,5 | 0,5 | 0,5 |
| Glycerin | 2,5 | 1,5 | 2,5 | 3,5 | 2,5 |
| Kaliumhydroxid | 0,2 | 0,2 | 0,4 | 0,2 | 0,2 |
| Hydroxypropylmethylcellulose | 0,15 | 0,15 | 0,25 | 0,15 | 0,15 |
| Ginkgoextrakt | 0,4 | 0,4 | 0,4 | 0,4 | 0,4 |
| Coco-Caprylate/Caprat | 0,5 | 0,5 | 0,5 | 0,5 | 0,5 |
| Triethanolamin | 3,1 | 3,1 | 3,1 | 3,1 | 3,1 |
| PEG-7M | 0,5 | 0,75 | 1,5 | 0,35 | 0,5 |
| Carnitin | 1,0 | 3,0 | 4,0 | 0,5 | 2,0 |
| Licochalcone A | 0,35 | 0,35 | 0,35 | 0,35 | 0,35 |
| Decandiol | 0,3 | 0,3 | 0,6 | 0,15 | 0,3 |
| Parfum | 0,3 | 0,3 | 0,45 | 0,3 | 0,3 |

## Patentansprüche

1. Kosmetische oder dermatologische Zubereitung umfassend 1,2-Decandiol, Licochalcone A und Carnitin, **dadurch gekennzeichnet, dass** der Anteil an 1,2-Decandiol im Bereich von mehr als 0,1 Gew.%, bezogen auf die Gesamtmasse der Zubereitung, gewählt wird.

2. Zubereitung nach Anspruch 1, **dadurch gekennzeichnet, dass** der Anteil an 1,2-Decandiol im Bereich von mehr als 0,2 Gew.%, bezogen auf die Gesamtmasse der Zubereitung, gewählt wird.

3. Zubereitung nach einem der vorstehenden Ansprüche, **dadurch gekennzeichnet, dass** der Anteil an Licochalcone A im Bereich von 0,025 bis 1 Gew.%, insbesondere im Bereich von 0,5 Gew.%, bezogen auf die Gesamtmasse der Zubereitung, gewählt wird.

4. Zubereitung nach einem der vorstehenden Ansprüche, **dadurch gekennzeichnet. dass** der Anteil an Carnitin im Bereich von 1 bis 5 Gew.%, bezogen auf die Gesamtmasse der Zubereitung, gewählt wird.

5. Zubereitung nach einem der vorstehenden Ansprüche, **dadurch gekennzeichnet, dass** Milchsäure zu einem Anteil im Bereich von 1 bis 5 Gew.%, bezogen auf die Gesamtmasse der Zubereitung, enthalten ist.

6. Zwei oder mehrere Zubereitungen nach einem der vorstehenden Ansprüche als Systemkomponenten zur Hautpflege oder-behandlung, **dadurch gekennzeichnet, dass** mindestens zwei der Zubereitungen räumlich benachbart angeordnet sind und/oder deren Anwendung auf der Haut zeitlich aufeinander abgestimmt ist

7. Verwendung der Kombination aus 1,2 Decandiol, Licochalcone A und Carnitin zur Herstellung eines Mittels zur therapeutischen Behandlung von Akne.

## Claims

1. Cosmetic or dermatological preparation comprising 1,2-decanediol, licochalcone A and carnitine, **characterized in that** the fraction of 1,2-decanediol is selected in the region of more than 0.1% by weight, based on the total mass of the preparation.

2. Preparation according to Claim 1, **characterized in that** the fraction of 1,2-decanediol is selected in the region of more than 0.2% by weight, based on the total mass of the preparation.

3. Preparation according to one of the preceding claims, **characterized in that** the fraction of licochalcone A is selected in the range from 0.025 to 1% by weight, in particular in the region of 0.5% by weight, based on the total mass of the preparation.

4. Preparation according to one of the preceding claims, **characterized in that** the fraction of carnitine is selected in the range from 1 to 5% by weight, based on the total mass of the preparation.

5. Preparation according to one of the preceding claims, **characterized in that** lactic acid is present in a fraction in the range from 1 to 5% by weight, based on the total mass of the preparation.

6. Two or more preparations according to one of the preceding claims as system components for skin care or skin treatment, **characterized in that** at least two of the preparations are arranged spatially adjacent and/or the application thereof to the skin is temporally coordinated.

7. Use of the combination of 1,2-decanediol, licochalcone A and carnitine for producing an agent for the therapeutic treatment of acne.

## Revendications

1. Préparation cosmétique ou dermatologique comprenant du 1,2-décanediol, du Licochalcone A et de la carnitine, **caractérisée en ce que** la proportion de 1,2-décanediol est choisie dans la plage de plus de 0,1 % en poids par rapport à la masse totale de la préparation.

2. Préparation selon la revendication 1, **caractérisée en ce que** la proportion de 1,2-décanediol est choisie dans la plage de plus de 0,2 % en poids par rapport à la masse totale de la préparation.

3. Préparation selon l'une quelconque des revendications précédentes, **caractérisée en ce que** la proportion de Licochalcone A est choisie dans la plage de 0,025 à 1 % en poids, en particulier dans la plage de 0,5 % en poids par rapport à la masse totale de la préparation.

4. Préparation selon l'une quelconque des revendications précédentes, **caractérisée en ce que** la proportion de carnitine est choisie dans la plage de 1 à 5 % en poids par rapport à la masse totale de la préparation.

5. Préparation selon l'une quelconque des revendications précédentes, **caractérisée en ce que** de l'acide lactique est contenu dans une proportion dans une plage de 1 à 5 % en poids par rapport à la masse totale de la préparation.

6. Deux ou plusieurs préparations selon l'une quelconque des revendications précédentes en tant que composants de systèmes pour le soin ou le traitement de la peau, **caractérisées en ce qu'**au moins deux des préparations sont disposées spatialement l'une à côté de l'autre et/ou leur application sur la peau est ajustée l'une à l'autre dans le temps.

7. Utilisation de la combinaison de 1,2-décanediol, de Licochalcone A et de carnitine pour la préparation d'un agent de traitement thérapeutique de l'acné.
